# EUROPEAN PATENT APPLICATION

(11) **EP 3 910 052 A1**
(43) Date of publication of application: **17.11.2021**
(21) Application number: 20174469.5
(22) Date of filing: 13.05.2020
(51) Int. Cl.: C12M 1/00

(54) **MAGNETIC CLEAVAGE OF COMPOUNDS**

(71) Applicant: PreOmics GmbH, 82152 Planegg/Martinsried (DE)
(72) Inventor: A. Kulak, Nils, 80686 München (DE); Hartinger, Katrin, 81249 München (DE); Käsemann, Martin, 40789 Monheim (DE); Johansson, Sebastian, 82284 Grafrath (DE); Wächter, Jasmin, 82284 Grafrath (DE)
(74) Representative: Dentons Patent Solutions Rechtsanwaltsgesellschaft mbH

(57) **Abstract**

This invention provides a method of cleaving at least one covalent bond in at least one molecule, wherein said method comprises (a) colliding at least one magnetic body with said at least one molecule; and/or (b) triggering collision of at least one nonmagnetic particle with said at least one molecule by moving said at least one magnetic body. In embodiments, the molecule is in suspension or solution and (a) the solution or suspension is a liquid sample such as a buffer or a bodily fluid, and the molecule is a nucleic acid; (b) the solution or suspension is drinking water or pool water, and the molecule is a macromolecular contamination; (c) the solution or suspension is sewage, and the molecule is a biological macromolecule; (d) the solution or suspension is food or beverage, and the molecule is gluten; (e) the solution or suspension comprises a protein, polypeptide and/or peptide, proteinaceous raw material for growth media and/or proteinaceous raw material for a personal care product such as a shampoo, a conditioner or soap, and the molecule is said or a protein, polypeptide and/or peptide; (f) the solution or suspension comprises a macromolecular toxin, and the molecule is said macromolecular toxin; or (g) the solution or suspension comprises undesired enzymatic activity, and the molecule is the protein exhibiting said enzymatic activity.

## Description

The present invention relates to a method of cleaving at least one covalent bond in at least one molecule, wherein said method comprises (a) colliding at least one magnetic body with said at least one molecule; and/or triggering collision of at least one non-magnetic particle with said at least one molecule by moving said at least one magnetic body.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

A number of industrial processes require cleavage of larger molecules because these molecules have undesirable properties whereas their cleavage products are acceptable or even have desirable properties. Examples of such larger molecules include molecules of biological origin ranging from proteinaceous molecules to carbohydrates and nucleic acids. Exemplary processes include the removal of unwanted enzymatic activity by cleaving the enzyme conferring said activity, the detoxification of compositions comprising a toxic protein where the cleavage product of the toxin does not anymore exhibit toxic activity; and production of nucleic acid-free reagents such as buffers, in particular free of long nucleic acids.

In many instances, enzymes are used for these cleavage processes. However, these enzymes have to be manufactured or obtained in large quantities, given that the mentioned processes are routinely performed on a large scale. Not only is enzyme production as such an involved process, but it may also entail a risk of contamination, e.g. when living cells are employed for production or the enzyme needs to be purified from animal sources. Also, while enzymes in many instances are still the first choice for the described cleavage processes, it has to be noted that this means that yet another agent of biological origin is used for diminishing or abolishing unwanted activity of a biological molecule. Also, enzymes are delicate agents - typically retain activity only under very strictly defined conditions and modifications, such as oxidation or denaturation due to pH or heat, may cause loss of function.

The use of magnets is not alien to the processing of molecules of biological origin and compositions in general. A very familiar use is a magnetic stirring bar which rotates under the influence of an external magnetic field and provides for rapid mixing of liquids, solutions and suspensions.

In view of the shortcomings described above, there is a need for improved means and methods of cleaving molecules with an unwanted activity, wherein said means and methods do not require use of yet another chemically or biologically active agent.

The technical problem underlying the present invention can therefore be seen in the provision of such means and methods, more specifically non-chemical and non-enzymatic means and methods of cleaving or fragmenting chain molecules and macromolecules, including those of biological origin.

This technical problem is solved by the subject-matter of the claims and the aspects and embodiments disclosed in the following.

More specifically, in a first aspect, the present invention provides a method of cleaving at least one covalent bond in at least one molecule, wherein said method comprises (a) colliding at least one magnetic body with said at least one molecule; and/or (b) triggering collision of at least one non-magnetic particle with said at least one molecule by moving said at least one magnetic body.

The term "cleaving" refers to breaking a covalent bond such that fragments of the starting molecules are formed. Cleavage may directly lead to stable fragments or to intermediate reactive species which in turn react with other molecules present to yield stable products. Other molecules may include water or more generally speaking, any molecules which are capable of forming stable adducts with reactive species to the extent they are formed. The terms "cleaving" and "fragmenting" are used equivalently herein as are the terms "cleavage products" and "fragments".

The covalent bond that is cleaved is not particularly limited. Included are single bonds as well as double and triple bonds, which all may between atoms of the same type (such as a C-C bond) or different atoms (such as a C-N bond or a C-O bond). Preferred are bonds occurring in functional groups linking building blocks of macromolecules such as peptide bonds, ester bonds including phospho-esters and glycosidic bonds. It is understood that such preferred bonds generally have a lower binding energy as compared to, e.g. a C=C bond, and are therefore amenable to cleavage with the method of the invention when operated in a manner which transfers less energy to the reaction mixture.

As will become apparent in the following, the present invention allows to control the amount of energy transferred to the material being processed, thereby providing means to target covalent bonds depending on their binding energies.

The number of fragments obtained from a given molecule is not particularly limited and may vary between two fragments and any higher number such as 3, 4, 5, 6, 7, 8, 9, 10, 20, 50, 100, 200, 500, 1000 or more fragments. Owing to the non-enzymatic and non-chemical cleavage process of the invention, fragmentation patterns may be generated which previously were not attainable. In this context, the term "fragmentation pattern" refers to both the sites of fragmentation as well as the average size and size distribution of the fragments obtained from a given molecule.

As regards the size of the obtained fragments, this is not particularly limited either. The size may preferably be controlled by the parameters controlling the method as disclosed further below (frequency, amperage, power, magnetic flux density etc.). The size of fragments may range from 1, 2, 3, 4, 5, 6, 7, 8, 9, 10 or any other two-digit number of atoms up to fragments which are comparable in size to said molecule, e.g. in those cases where only one, two or a single-digit number of moieties is cleaved off which are small in comparison to said molecule.

The term "at least one molecule" embraces applications which use exactly or approximately one molecule, also termed "single molecule applications". Where there is no need to study or handle individual molecules, but to fragment small numbers of molecules, amounts from 1 fmol to 1 µmol are embraced by the term "at least one molecule". Also embraced are macroscopic amounts, e.g. in the range from 1 µmol to 10⁶ mol. The latter applications are preferred, also in view of preferred embodiments detailed further below which include processes routinely performed on a large scale in the field of processing water, beverages and food. Especially in the context of applications of the invention in flow-through reactors, there is virtually no limit on the amount to be processed - it may well exceed 10⁶ mol, such as 10⁷ to 10¹⁰ mol. Indeed, in the context of such continuous processes (for details see further below), the absolute amount is not particularly limited. For example, flow rates from 1 mol per hour up to 10⁶ or 10⁷ mol per hour are envisaged.

It is understood that when performing the method of the invention, other molecules in addition to the molecules to be cleaved may be, and generally will be present.

Moreover, the molecules to be cleaved may belong to a single molecular species or may form a family of more or less closely related molecules, such as a polyclonal antibody.

Both types of mixtures of the molecule under consideration - with molecules to be cleaved as well as with molecules cleavage of which is not required or to be avoided - are envisaged.

The term "colliding" refers to a relative motion between said magnetic body and said molecule. Collisions may occur more than once. Colliding will generally entail that magnetic body and molecule encounter in close contact. Under such circumstances of close spatial proximity, a transfer of energy between the moving magnetic body (or a moving non-magnetic particle; see in the following) is possible and confers the energy required for bond cleavage to said molecule.

In an alternative or in addition, e.g. where a plurality of magnetic bodies is used and/or one or more non-magnetic particles (for details see further below) are present, the motion of a given magnetic body may act as a trigger of collisions of a molecule with another magnetic body or a non-magnetic particle. In other words, a given magnetic body may collide with a given molecule and/or cause collision of said molecules with other magnetic bodies or particles, wherein either type of collision is capable of triggering cleavage of a covalent bond in said molecule. The latter type of collision is also referred to as "indirect" effect of the motion of said magnetic body and is defined by item (b) of the method of the first aspect. Options (a) and (b) will generally occur together when at least one magnetic body and at least one non-magnetic particle of bead are present. Yet, fragmenting via a purely indirect effect is envisaged as well.

In addition to the cleavage of covalent bonds, the method of the invention may also be used to interfere with other types of intra- and intermolecular interactions such as van der Waals interactions, hydrogen bonds and hydrophobic interactions.

The method may be performed with a single magnetic body which may be implemented as a single magnet, i.e., a piece of ferri-, ferro- or paramagnetic material. Alternative implementations of the magnetic body are disclosed further below.

As an alternative to said single magnet or single magnetic body, a plurality of magnetic bodies or magnets may be used. The number of magnetic bodies is not particularly limited, although preference is given to a single-digit or double-digit number of magnetic bodies, such as exactly 2, 3, 4, 5, 6, 7, 8, 9, 10, 15, 20, 25, 30, 40 or 50 magnetic bodies or magnets. It is understood that the number of magnetic bodies, to the extent it is specified, is limiting. In other words, while the method of the first aspect may comprise other measures, such possibility does not extend to the option of more magnetic bodies being present than expressly specified.

Usually the method of the invention will be practiced in a defined space, e.g. within a reactor. Said space may also be defined by the total volume of a solution or suspension, to the extent said at least one molecule is in solution or suspension.

Under such circumstances the number of magnetic bodies may also be defined indirectly in terms of their total volume. Preferably said total volume occupied by all magnetic bodies together is between 0.01% and 98% of the volume of the reactor, the volume of the solution, or the volume of the suspension, more preferably between 0.1% and 10%.

The structure and origin of the molecules to be cleaved are not limited. Preference is given to macromolecules of biological origin; for details see below.

The present inventors surprisingly discovered that a rapidly moving magnet is capable of triggering fragmentation of macromolecules. Of note, such fragmentation occurs in the absence of any established chemical or biologicals known to cleave a given macromolecule. This unexpected possibility is also referred to as "magnetically induced cleavage" herein, or, when applied to proteins, "magnetically induced proteolysis".

This unprecedented finding opens the door to a range of applications which constitute preferred embodiments of this invention and are described in more detail further below. What is common to these applications is that an important deficiency of art-established processes is overcome. To explain further, up to date an unwanted activity originating from a first biological molecule is often reduced or abolished by adding a further molecule of biological origin to a reaction mixture comprising the unwanted activity. While such further molecule of biological origin, e.g. an enzyme, has the advantageous property to diminish or abolish the unwanted activity by cleaving or catalysing cleavage of the first biological molecule, it may in turn present an eventually undesirable contaminant of the obtained product. This is aggravated by the fact that many enzymes, owing to their method of manufacture, are not 100% pure but comprise further constituents which may have deleterious or immunogenic properties.

In addition, manufacture of enzymes is generally an involved process entailing substantial cost.

The invention is not limited to the removal or inactivation of unwanted molecules or activities. There are also applications where the focus is on the products of cleavage, for example where the fragments or one of the fragments obtained are compounds of interest, be it in terms of active agents (this term not being confined to drugs), pharmaceuticals, diagnostics or for analytical purposes.

The method of the present invention overcomes these deficiencies or addresses such needs, respectively. There is no longer a requirement for a chemical or biological agent for cleaving a macromolecule. The method is easy to implement. Exemplary or preferred equipment, devices and kits for that purpose are also subject of this invention and described further below. In an exemplary fashion, the method has been applied for the purpose of fragmenting nucleic acids; see Example 1.

In a preferred embodiment, said at least one magnetic body performs a fluctuating or oscillating motion.

For example, the magnetic body moves up and down and back and forth, wherein the motion may have regular or repeating components but does not have to, and wherein spatial directions are not particularly limited. Also, the magnetic body may rotate about one or more axes, usually in addition to translational motion. For a more precise description of the envisaged types of motion, see further below. To the extent a reactor, i.e. a container, enclosure or vessel is used, the magnetic body may, but does not have to, hit or repeatedly hit a wall of said reactor. Accordingly, the magnetic body, while being in motion all the time (if specific implementations of the method of the invention do not specify otherwise, e.g., an intermittent motion), such motion is generally not a directed motion. Also, said motion, despite being possibly irregular, generally is about an average position which is located within the mentioned enclosure or container - the magnetic body does not leave the reactor. The motion of the magnetic body generally has one or more translational components; and said average position may be somewhere in the middle of said reactor. As such, the motion is different from the motion performed by a magnetic stirrer - which is a rotation, and the average position of the magnet is at or close to the bottom of the vessel containing the liquid to be mixed or stirred.

The term "oscillating" designates a regular motion, whereas the term "fluctuating" is broader and embraces also irregular motion. There is no particular preference in that respect. In practice, given that often the magnet not only collides with the molecules to be cleaved, but also with at least one wall of a reactor or enclosure used to contain the magnetic body and the molecule, irregular motion occurs more often. In either case, the motion ensures that the magnetic body is brought into contact with said molecule, generally repeatedly, and also repeatedly with all or substantially all molecules to be cleaved to the extent a plurality of molecule or a macroscopic amount thereof is to be processed by the method of the invention. As mentioned above, the motion of said magnetic body might also cause collisions of said molecule with further magnetic bodies and/or non-magnetic particles, to the extent they are present. In such cases, the action of a given magnetic body on a covalent bond could also be termed "indirect" as opposed to a "direct" collision between said given magnetic body and a molecule. Importantly, such indirect action is sufficient to trigger cleavage, i.e., collision between the magnetic body and the molecule is not required.

In other words, in a related aspect, the present invention provides a method of cleaving at least one covalent bond in at least one molecule, wherein said method comprises moving at least one magnetic body relative to said at least one molecule in the presence of non-magnetic beads.

For preferred embodiments of non-magnetic beads, see further below. The size of said non-magnetic beads may be between the mass of the molecule to be cleaved and the mass of said at least one magnetic body.

It is understood that said relative movement of said magnetic body relative to said molecule in the presence of non-magnetic beads will trigger collisions between said molecule and said non-magnetic particles. Obviously, collisions between said magnetic body and said molecule, i.e., "direct collisions", may also occur in such a setting, but there is no requirement in that respect.

Preferably, said at least one magnetic body performs a fluctuating or oscillating motion.

In a preferred embodiment of any of the above disclosed aspects of this invention, said motion is triggered by a fluctuating or oscillating magnetic field.

A magnetic field is a common means of controlling position and/or motion of a magnet. Given that in accordance with the invention, the magnetic body moves, use is made of a fluctuating or oscillating magnetic field in this preferred embodiment. Any such magnetic field may be useful.

Preference is given to said magnetic field being generated by an electric current. It is well established that electric and magnetic fields are interrelated; in particular that an electric current generates a magnetic field. As a consequence, controlling the electric current is a means of controlling the magnetic field generated thereby.

In an alternative or in addition, said magnetic field may be generated or modulated, respectively, by an external magnet. The term "external" means that such magnet is not located within said reactor. The external magnet may be a permanent magnet. In case of using an external magnet, the fluctuating or oscillating motion of said magnetic body is triggered by a corresponding movement of said external magnet relative to said magnetic body.

In a preferred embodiment, said magnetic field is generated by an electromagnet. As used herein, the term "electromagnet" embraces, in its simplest implementation, a piece of an electric conductor through which an electric current is flowing when in use. For better control of the magnetic field or for the purpose of generating stronger magnetic fields, particular implementations of the electromagnet are envisaged which are subject of preferred embodiments disclosed further below.

In a preferred embodiment, said electric current fluctuates or oscillates. This behavior may also be referred to as a generic "wave". The amount of an electric current is known as amperage.

In a preferred embodiment, amperage of said electric current as a function of time is (i) a rectangular function; (ii) a sinusoidal function; (iii) a triangular function; (iv) a sawtooth function; or (v) a combination or convolution of any one of (i) to (iv).

Given that the electric current oscillates or fluctuates, this also applies to patterns (i) to (v), i.e., said rectangular and said triangular functions are in fact repeating rectangular and triangular functions. The term "pattern" designates a series of events where a given basic event is repeated at least once. In a wider sense, repetition does not have to be a precise repetition - the lengths of e.g. rectangles in a time graph may change (which effectively amounts to a change of frequency, preferred frequencies as well as preferred time dependencies of frequencies being specified further below).

All embodiments (i) to (v) are also referred to as "alternating current" herein.

Particularly preferred is said rectangular function (as referred to as rectangular wave or square wave), more specifically the patterns of repeating rectangular functions. The inventors surprisingly found that this pattern triggers particularly vigorous motion of the magnetic body, wherein such vigorous motion is particularly efficient in terms of cleavage. In said rectangular function, the time intervals of high current and low current (or the current being off) may be the same or different. Means to control the length of said time intervals are known to the skilled person, e.g. those referred to as pulse width modulation (PWM). Of note, the energy transferred to the reaction mixture is not only governed by frequency and amplitude of the electric current, but also by the relative duration of said time intervals.

In a further preferred embodiment of the method of the first aspect, said colliding transfers an amount of energy to said molecule which is sufficient to cleave at least one covalent bond. It is well established that collision is a means of transferring energy from one body to another. The energy received by the receiving body may be converted into internal energy of said body (here a molecule) and trigger its fragmentation.

Energies contained in covalent bonds are known; these energies also define the energy required for cleavage, especially in the absence of a catalyst. Most covalent bond energies fall into the interval from 100 to 1200 kj/mol; see, e.g., Chemistry: Atoms First 2e, ISBN 978-1-947172-63-0. To give a few examples of covalent bond energies, a C-C bond as a bond energy of 345 kj/mol, a C-N bond of 290 kj/mol, and a C-O bond of 350 kJ/mol.

The above disclosed preferred embodiment is a means to specify the overall result of a specific implementation chosen: The energy transferred to the molecule to be cleaved shall be such that at least one covalent bond in said molecule receives the amount of energy required for dissociation.

As an estimate, the amount of energy transferred by the magnetic body to a covalent bond is equal to or less than the energy transferred by the magnetic field to the magnetic body. The energy in a magnetic field per unit volume is defined by *E_{mag}* = ½ *B*²/*µ₀;* for definitions of B and *µ₀* see further below. *E_{mag}* in turn is equal or less than the energy of the electric current which causes the magnetic field. The latter energy can be estimated as *E_{curr}* = *U I t, U* being the voltage and *I* the amperage of the current generating the magnetic field, and t is the time during which the current flows. In other words, controlling any one of *B, U, I* and *t* is a means of controlling the amount of energy transferred by the magnetic body to a covalent bond.

Having said that, there are other means of specifying the detailed implementation of the method of the first aspect. This includes specifying one or more of a plurality of parameters which can be more directly controlled or measured. These parameters include frequency and amperage of the current and may furthermore include dimensions of a reactor and a coil, to the extent use is made thereof. Also, the strength of the magnetic field, preferably at the site of the magnetic body, is a means of quantitatively specifying implementation details. In all these cases, preference is given to those parameter values or combinations of parameter values which ensure that the above requirement (transfer of sufficient energy to cleave a bond) is met. In the following, preferred ranges of the mentioned parameters are specified.

In a preferred embodiment, said electric current fluctuates or oscillates with a given frequency, preferably a frequency of 0.1 Hz to 20 MHz, more preferably 10 Hz to 2 kHz, yet more preferably 50 to 500 Hz or 90 to 300 Hz or 100 to 200 Hz. It is understood that these measures apply not only to sinusoidal current, but to all current profiles specified herein, including, e.g., the repeated rectangular pattern. The term frequency may also apply to fluctuations, i.e., time-dependent behavior which is not regular (such regular time behavior also referred to as "oscillation" herein) and is a means to characterize the timescale of fluctuations. In such a case, the term "frequency" is understood as referring to the average frequency of the fluctuation.

It turns out that at least for reactors with a volume in the one-digit to two-digit mL range as well as for the wells of a standard 96-well plate, lower frequencies between 80 and 300 Hz work particularly well, whereas significantly higher frequencies such as around 1000 Hz, while inducing vibration of the magnetic body, might not trigger the full range of motion which covers a significant portion of the volume of the reactor. This does not mean that higher frequencies are not beneficial. Also, they may be used in conjunction with lower frequencies (see below).

More generally speaking, a preferred frequency range is a range which ensures that the magnetic body not only vibrates or rotates, but performs a translational motion which explores the entire volume or substantially the entire volume of the material to be processed with the method of the invention. In those implementations which make use of a reactor and the material to be processed is in solution or suspension, said volume is the total volume of said solution or suspension as contained in said reactor. In other words, while guidance is given above with regard to reactors with a volume in the one-digit to two-digit mL range and 96-well plates, the frequency ranges may need adaptation for reactors with significantly smaller volume, significantly larger volume, or special geometries. To give an example, it is expected that for smaller volumes such as the wells of high-density microtiter plates (e.g. 1536-well plates) higher frequencies, e.g. of about 1 kHz, lead to a motion of the magnetic body which is comparable to the motion seen in larger vessels at lower frequencies. In any case, a skilled person provided with the guidance given in this specification, can explore and optimize in a straightforward manner the parameters controlling motion of said at least one magnetic body. As explained further below, preference is given to the magnetic body performing translational motion, preferably in addition to rotation.

In a further preferred embodiment, said method comprises raising the temperature of said magnetic body. This is an effect which is to some degree inherent to the use of a magnetic field to trigger motion of said magnetic body. It also known as induction heating. The rise in temperature may occur predominantly on the surface of the magnetic body (also referring to as "skinning" in the art) or may affect the entire magnetic body. Frequency (for preferred ranges see above) is a means to control the extent to which temperature rises and/or where it predominantly occurs in the magnetic body. To give an example, if a heating of the surface of the magnetic body is desirable, frequencies in the range from 10 kHz to 20 kHz are preferable. This frequency range preferably is applied in addition to the frequencies preferred for fragmenting.

In a preferred embodiment, said frequency is kept constant throughout while said method is performed.

In an alternative preferred embodiment, said frequency changes as a function of time.

In a further preferred embodiment, more than one frequency is applied at a given point in time. In such a case, each frequency of such a plurality of frequencies may be chosen from any of the preferred intervals given above. Particularly preferred in case of two frequencies is that the first frequency is between 50 Hz and 500 Hz and the second frequency between 80 Hz and 20 MHz. In other words, this preferred embodiment provides for the superposition of a plurality of frequencies.

More than one frequency includes 2, 3, 4, 5, 6, 7, 8, 9 and 10 different frequencies. Such plurality of frequencies may be applied throughout in place of a single frequency - which means that they are applied during the entire performance of the method. Also, a plurality of frequencies, or different pluralities of frequencies may be applied in different time intervals within a longer time span. Within said longer time span, and in addition to time intervals where more than one frequency is applied, there may be one or more, such as 2, 3, 4, 5, 6, 7, 8, 9, or 10 time intervals where only one frequency is applied.

The inventors surprisingly found that a regime where more than one frequency is applied performs superior in terms of yield. "Yield" designates the relative quantity of fragmented molecules compared to the total number of (unfragmented) molecules at the beginning of the method.

In a further preferred embodiment, said frequency is, and, in case more than one frequency is applied, the frequencies are not constant over time, and preferably is/ are switched or gradually changed between two or more frequencies, preferably in a periodic manner.

Exemplary regimes are (120 Hz -1000 Hz)ₙ, (200 Hz -1000 Hz)ₙ, or (100 Hz - 800 Hz)ₙ, wherein n is an integer, e.g. between 2 and 1000, such as between 10 and 100, and specifies the number of times the frequency pattern in brackets is to be repeated.

The duration of the time interval with constant frequency and / or constant amperage is not particularly limited. Envisaged are time intervals between 1 sec and 1 day, such as between 1 min and 1 hr.

In a further preferred embodiment, said electric current (a) has an amperage I between 20 mA and 100 A, preferably between 0,1 and 20 A; (b) exposes said magnet to a magnetic field strength between 0,02 and 10⁹ A/m, preferably between 10 and 10⁶ A/m; and/or (c) is applied for a time span t between 1 sec and 1 week, such as between 10 min and 5 hrs.

Keeping in mind that, as disclosed above, the amperage as a function of time fluctuates on a timescale governed by the frequencies disclosed herein, there is no constant amperage on the timescale of the fluctuation. Yet, for practical purposes, and in line with established practice in electrodynamics, an alternating current may be quantified in terms of its average amperage. The above values are average amperages in that sense. Of note, the average is preferably over the time scale of the fluctuations. That means, to the extent intermittent current is used, there will be an average amperage, preferably within the ranges specified above, when the current is on, and there will be zero amperage when the current is off.

The magnetic field strength H determines the intensity of the field and is measured in A per meter. H has to be distinguished from the magnetic flux density B which is particularly relevant in setting where a core is used to re-enforce the magnetic field of an electric current; see below.

In a preferred embodiment, the amplitude of fluctuation or oscillation is (a) constant; or (b) changes over time, preferably on a timescale which is slower than the timescale of said fluctuation or oscillation.

This embodiment refers to the amplitude of motion of said electric current. The amplitude of oscillation or fluctuation of an electric current is governed by the amperage.

In a further preferred embodiment, said current is intermittent and/or said amperage changes over time, preferably in a periodic manner. This change over time is generally on a time scale which is slower than the time scale defined by the frequency of the alternating current. In other words, if an alternating current changes over time in the sense of this embodiment, the time dependency of the current is a superposition of two patterns or waves: a generally fast fluctuation which is inherent to an alternating current, and a generally slower change.

An exemplary intermittent pattern is a repetition of the sequence on (1 min) - off (1 min). Other preferred time intervals are given above. Advantages of intermittent patterns allow for keeping temperature constant or substantially constant, especially if it is observed that the contents of the reactor is heating up.

In a further preferred embodiment, a power of between 0 and 1000 W, preferably between 1 and 200 W is applied.

In a further preferred embodiment, the electric current is powered by an electric power source. Preferably, the electric power source has an electric potential or voltage U in the range between about 0 and 240 V such as between 1 and 75 V. These values refer to the mean voltage applied.

In a further preferred embodiment, said electromagnet comprises at least one coil, wherein preferably said coil (a) has a plurality of or windings, such as between 1 and 10⁴, preferably between 10 and 1000; and/or (b) comprises at least one Helmholtz coil; and/or (c) comprises at least one core.

Exemplary numbers of a plurality of coils are 2, 3, 4, 5, 6, 7, 8, 9, 10, 20, 50, 100, 150, 200, 300, 400 and 500.

The term "Helmholtz coil" is established in the art and refers to an arrangement of typically two identical coils spaced apart such that their axes of rotational symmetry align or coincide. The magnetic field in the space between the coils is particularly homogenous and/or particularly strong. Arrangements of at least one Helmholtz coil may be two Helmholtz coils.

Further arrangements of a plurality of coils are known to provide for extended spatial regions of particularly homogeneous magnetic field. A further example is a Maxwell coil.

A core serves to re-enforce the effect of the magnetic field. A core is preferably made of ferromagnetic material such as iron, in particular soft iron. The magnetic flux density B is related to magnetic field strength as follows: *B* = *µᵣµ₀ H. µ₀* is the magnetic permeability of vacuum and as such a fundamental physical constant. *µᵣ* on the other hand is the relative permeability and determines the degree of re-enforcement of the magnetic flux density by a given material, e.g. a ferromagnetic core when under the influence of a magnetic field. Typically, µᵣ for ferromagnetic materials to be used as a core is between 10³ and 10⁶ such as between 200000 and 400000, e.g. about 300000. Suitable core materials comprise powdered metals, laminated metals, annealed metals such as annealed iron, ceramics, and solid metals.

Preferred values of B in the absence of a core are between 10⁻⁸ and 10⁴ Tesla (T), such as between 10⁻⁵ and 1 T. If a core with a specific relative permeability is used, the values of B are to be multiplied with said relative permeability. As such, preferred values of B in the presence of a core are between 10⁻² and 10⁹ such as between 1 and 10⁶ T. Generally, these values refer to B at the site of the magnetic body or within said reactor.

In terms of geometry, preferred coils are circular. Preferred diameters are between 1 mm and 1 m or 1 mm and 0,5 m, such as between 2 mm and 300 mm or 2mm and 200mm. Envisaged are also different geometries such as coils with a square, a rectangular or a triangular shape (i.e., all or part of the windings are a square, a rectangle or a triangle). Finally, and noting that a coil is not an indispensable requirement for an electromagnet, also an arrangement of two antiparallel wires may be used ("antiparallel" referring to the direction of the electric current flowing through the two wires at a given point in time).

In a further preferred embodiment, more than one coil is used, preferably between 2 and 10⁴ such 2,3,4,5, 6, 7,8, 9, or 10 coils, or between 10 and 1000 coils. It is understood that each coil may have one or a plurality of windings, preferred numbers of windings being disclosed herein above.

In a further preferred embodiment, said magnetic body is (a) a single magnet; or (b) an assembly of particles at least one of which is a magnet and assembly of said particles is mediated by magnetic fields of said at least one magnet.

In the simplest arrangement, said at least one magnetic body is implemented by a single magnet. Sizes of single magnets may vary widely and may be appropriately chosen in dependency of the dimension of the reactor or vessel to be used. As regards this relative size criterion (size of magnet vs. size of vessel), see further below. To the extent present, other material in the reactor or reaction mixture may be taken into account when choosing the size of the magnetic body, such other material including further components of the sample in addition to the molecule(s) to be cleaved and/or non-magnetic beads.

For the sake of completeness, exemplary values of the size of a useful magnet are given here to be between 0.1 mm and 10 cm such as between 0.2 mm and 2 cm, including any of the following values and ranges defined thereby: 0.3 cm, 0.4 cm, 0.5 cm, 0.6 cm, 0.7 cm, 0.8 cm, 0.9 cm, and 1 cm. The same preferred sizes and size ranges apply to any magnetic body considered herein, also to implementations where use is made of a plurality of magnetic bodies and/or magnets. Generally, these lengths refer to the largest extension of said magnetic body.

Alternatively, a plurality of magnets such as those defined above may be used. Exemplary, but non-limiting numbers are in the one-digit and two-digit range such as 2, 3, 4, 5, 6, 7, 8, 9, and 10. Also more than 10 such as 20, 30, 40, or 50 magnets may be used. These numbers refer to one reactor or vessel, also in case arrangements of vessels or reactors (such as microtiter plates) are used.

In terms of shape of said magnet, there are no particular limitations, wherein preference is given to those shapes which do not negatively interfere with the free motion of the magnet. Exemplary shapes include sticks, bars, rods, rods with rounded ends, cubes, cuboids, prisms, spheres, elongate and oblate ellipsoids, disks, tetrahedrons, octahedrons, dodecahedrons, and icosahedrons.

Such setup has still to be distinguished from the above disclosed "assembly". The latter refers to a setup where a number of particles (in the simplest case one particle or bead such as a ferromagnetic bead) assemble under the influence of a single or a few (such as tens) magnets (possibly re-enforced by the magnetic field in accordance with the invention) into a single magnetic body which essentially behaves like a single magnet. For the sake of completeness, also a plurality of magnetic bodies, each of them or part of them resulting from an assembly as described above, may be employed. Yet, this is not very likely under many circumstances. In particular, and in case no measures are taken to prevent this from happening, placing more than one assembly into the same reactor or vessel may lead to the formation of a single bigger assembly (which in turn also behaves like a magnet once in a magnetic field).

In a further preferred embodiment, (a) said magnet comprises or consists of ferromagnetic material or ferrimagnetic material; (b) the particles of said assembly as defined above comprise or consist of a material selected from ferromagnetic materials, ferrimagnetic materials, paramagnetic materials and/or diamagnetic materials; and/or (c) said magnet and/or said particles are coated, preferably with a coating selected from (i) a coating conferring chemical stability; (ii) a coating conferring mechanical stability or hardness; (iii) a coating with a catalyst; (iv) a coating with a nucleic acid such as a probe and primer; (v) a coating with a chelating agent such as IMAC, TiO₂ and ZrO₂; (vi) a coating with a chromatographic material, preferably selected from (1) reversed phase groups such as C18, C8, Benzene; (2) HILIC groups such as hydroxyl groups; (3) cation ion-exchange groups such as sulfonic acid, phosphoric acid, carboxylic acid; (4) anion-exchange groups such as primary, secondary, tertiary and quaternary amino groups; and (5) any combination of any one of (1) to (4); (vii) a coating with ligand-binding proteins and/or their cognate ligands, preferably selected from globulins, particularly immunoglobulins, streptavidin, biotin, Protein A, Protein G; enzymes such as oxidoreductases, transferases, ligases such as polymerases, hydrolases such as proteases, peptidases, nucleases, saccharidases, lipases, lyases, and isomerases; and (viii) a combination of any one of (i) to (vii).

Suitable materials for said magnets include the following elements and their alloys: neodymium-iron, neodymium-iron-boron (such as Nd₂Fe₁₄B), cobalt, gadolinium, terbium, dysprosium, iron, nickel, iron oxides, manganese-bismuth, manganese-antimony, manganese-arsenic, yttrium-iron oxides, chromium oxides, europium oxides, and samarium-cobalt. Particularly preferred materials are neodymium-iron and samarium-cobalt.

In a less preferred embodiment, what is referred to as "magnet" may also be implemented using paramagnetic material, in particular in case the magnetic susceptibility of such paramagnetic material is high.

Suitable coatings in accordance with (c)(i) include polypropylene, polyethylene, polystyrene, parylene, titanium nitride, polyimide, chloropolymers and fluoropolymers, preferably polytetrafluoroethylene (PTFE).

In a further preferred embodiment, said at least one molecule is in solution or in suspension. In such a case, the molecule to be fragmented is dissolved or suspended in a liquid phase and the magnetic body is located within said liquid phase (at for a significant proportion of the total time of the process in order to ensure cleavage).

Liquid handling is facilitated by containers. Accordingly, in a preferred embodiment, said at least one molecule and said at least one magnetic body are located in a reactor. The term "reactor" as used herein generically refers to an enclosure or container where said at least one molecule and said at least one magnetic body are located and allowed to interact ("react") in accordance with the invention. Otherwise, the term is not particularly limiting and embraces vessels, vessels with a closed bottom, vessels with a lid, vessels with a closed bottom and a lid, entirely closed or sealed vessels, tubular elements, and elements with at least two openings allowing for continuous liquid flow through an accordingly designed reactor. Reactors may also be implemented as microfluidic devices, i.e., miniaturized devices comprising one or more channels with openings, optionally with widenings or vessels and/or valves.

In a preferred embodiment, said method is performed in batch mode, e.g. by using a reactor which is a vessel with a closed bottom.

Exemplary and preferred vessels are those which are configured to hold a volume of 5 µL to 1 L, preferably between 10 µL to 50 mL, more preferably configured to hold volumes of any of 30 µL, 40 µL, 100 µL, 150 µL, 200 µL, 250 µL, 500 µL, 1 mL, 1.5 mL, 2 mL, 5 mL, 15 mL and 50 mL.

In a further preferred embodiment, said method is performed in continuous mode, e.g. said solution or suspension is allowed to flow over said at least one magnetic body, preferably (a) in a reactor with at least two openings; and/or (b) wherein motion of said magnetic body occurs while said solution or suspension is flowing.

Item (a) defines the reactor to be a tubular element with at least two openings. Cross-sections may vary widely depending on the specific application and the scale of the process performed. Accordingly, cross-sections between 0.1 mm and 1 m, such as between 0.5 mm and 10 cm, but also values outside these intervals are envisaged.

In terms of shape, and while a tubular element may be a simple and convenient implementation, also other shapes are envisaged, given that they provide for a flow-through. Such reactors may, and generally will, include means to control or modify flow-through of the solution or suspension comprising the molecule to be cleaved. In other words, flow-through is a further parameter in addition to the parameters controlling motion of the magnetic body which eventually govern performance, degree of cleavage and yield of the method of the invention.

In a further preferred embodiment, (a) the motion of said magnetic body is relative to said reactor; and/or (b) the average position of said at least one magnetic body is substantially constant relative to said reactor, preferably (i) owing to said magnetic field; and/or (ii) by a filter, a mesh, a membrane, or a sponge holding said magnetic body; or a thread, a filament, a wire, or a spring connecting said magnetic body to said reactor, preferably without significantly restricting said motion.

Item (a) merely specifies that the relative motion of magnetic body and molecule at the same time implies a relative motion of magnetic body and reactor, in the simplest case the same type of motion (i.e., when ignoring motion of the molecules relative to the reactor).

Item (b) provides means for preventing the magnetic body from jumping out of the reactor or being washed out of the flow-through reactor.

In a further preferred embodiment, the dimensions of said at least one magnetic body and said reactor are such that said at least one magnetic body moves freely or substantially freely about its average position.

As such, this embodiment has to be distinguished from designs for restricted motion where only a thin layer of liquid is allowed to pass through between magnet and inner wall of a vessel, e.g. a cylindrical magnet in a cylindrical vessel, the inner diameter of the vessel being only slightly larger than the (outer) diameter of the magnet.

As can easily be seen, the requirement of free motion is met for a magnet the largest extension of which is small as compared to the dimension or cross-section of the reactor, vessel or tube - such as for a 3 mm magnet in a 1.5 mL vessel. "Small as compared to the vessel" will generally mean that the largest dimension of magnet is smaller than the smallest dimension of the reactor, vessel or tube. In other words, the largest dimension of the magnet should fit through the smallest passage or cross-section in said reactor, vessel or tubular element. Smaller than the smallest dimension preferably means 2/3, ½, 1/3, ¼, or 10% of said smallest dimension or cross-section of said reactor, vessel or tube.

In a preferred embodiment, free or substantially free motion is around or along at least two, at least three, at least four, at least five or preferably all six axes of translational and rotational motion, and wherein preferably said free or substantially free motion includes translation along at least two axes.

For a point-like object, there are three degrees of motional freedom, i.e., translation in three independent directions spanning the three-dimensional space. For an extended object, there are three further degrees of freedom which can be defined in terms of three independent axes of rotation.

The above example - 3 mm magnet in a 1.5 mL vessel - is considered as an implementation of free of motion along or around all six axes. In particular, it includes freedom of motion along the recited at least two axes of translational motion and is as such clearly distinguished from the mention restricted motion design. Provided with the teaching of the specification, the skilled person can easily determine the most appropriate parameters of the method of the first aspect which provide for such motion; see, for example, the comment on frequencies further above.

In a further preferred embodiment, said at least one magnetic body collides with at least one wall of said reactor. This is not a necessity, but not a disadvantage. Collision may occur repeatedly. Of note, also in such embodiments, preference is given to free motion - like a 3 mm magnet in a 1.5 mL vessel repeatedly hitting the wall when traveling through the liquid phase contained therein.

In a further preferred embodiment, the inner diameter of said coil or of said at least one coil is such that said reactor or a plurality of reactors such as an array of reactors can be placed within said coil.

An exemplary implementation of this embodiment is a cylindrical vessel or a vessel with a cylindrical cross-section encircled by a coil.

Yet, also configurations with at least one coil above, below or adjacent to the reactor are envisaged and functional - what matters is that a magnetic field at the site of magnet is generated. There is also no requirement for the magnetic body to be located at the site of maximal magnetic field strength of magnetic flux density.

Similarly, and assuming the vessel would have a main axis of rotational symmetry, said axis may be aligned or even co-axial with the axis of symmetry of a coil, but does not have to. Any angle between the two axes may be used.

In a further preferred embodiment, said reactor has a circular cross-section and the inner diameter of said coil or of at least one coil is only slightly larger than the outer diameter of said reactor. Depending on the elements used and the precision in their manufacture, "slightly larger" can embrace, for example, a factor of 1.0001, 1.001, 1.01 or 1.1.

In a further preferred embodiment, at least a part of the wall of said reactor is magnetically permeable. More preferred is that the entire reactor is made of magnetically permeable material.

Suitable materials include plastic, polymers such as polypropylene, glass, and ceramics. Keeping the requirement of magnetic permeability in mind, also metals may be used.

At least one wall of the reactor may furthermore be coated or passivated, while paying attention to the requirement of magnetic permeability, e.g. with at least one material selected from with polypropylene, polyethylene, polystyrene, parylene, titanium nitride, copper, nickel, silver, chrome, epoxy-resin, zinc, tin, everlube, silica, phosphate, rubber, chloropolymers and/or a fluoropolymer preferably gold, titanium nitride, and polytetrafluoroethylene.

In a further preferred embodiment, said method is performed at (a) a temperature between -100 and 100 degree Celsius, preferably at ambient temperature such as about 20 degree Celsius; and/or (b) at a pressure between 0.1 and 10 bar, preferably at ambient pressure such as about 1 bar.

In other words, the invention can conveniently be put into practice under ambient conditions. Having said that, the precise conditions may depend or may be optimized dependent on the molecules to be cleaved. Modifying temperature and/or pressure may provide better performance as may the use of protective gas atmosphere.

In a further preferred embodiment, said at least one molecule is a macromolecule or a chain molecule, wherein said macromolecule or chain molecule comprises or consists of at least two building blocks, wherein preferably said building blocks are amino acids, nucleotides, ribonucleotides, sugars, saccharides, phosphates, fatty acids, glycerides, or a combination of any of the foregoing.

In other words, the term "molecule" embraces proteins, peptides, polypeptides, nucleic acids, saccharides and lipids. Nucleic acids include oligonucleotides and polynucleotides and may be or comprise RNA and DNA. Lipids include triglycerides, phosphatidyl compounds and sphingolipds. Molecules may be or comprise combinations of any of the above. To the extent the molecule is proteinaceous and of biological origin, it may be a protein with post-translational modifications, e.g. said protein may be N- and/or O-glycosylated, phosphorylated and/or prenylated.

The terms "macromolecule" and "chain molecule" are used to indicate that the molecule under consideration can be viewed as being made of a plurality of identical or related building blocks. The term "chain" furthermore implies a linear topology of the molecule, acknowledging that chain molecules of biological origin, while having a linear primary structure may assume defined secondary and tertiary structures - as is well known in the field of structural biology.

In terms of chemistry of the junction between building blocks, the molecules include polymers, polycondensates as well as combinations thereof. A polycondensate is a chain molecule formed from monomers wherein during formation, more specifically when the bond between two adjacent building blocks is formed, water is expelled. This applies, e.g., to polypeptides when they are formed from free amino acids as well as when they are assembled on a ribosome inside a living cell.

The molecular weight is not particularly limited, nor is the number of building blocks which can be as low as 2, 3, 4, 5, 6, 7, 8, or 9. More generally speaking, a typical range of the number of building blocks is between 10 and 100000, such as between 50 and 1000. Preferred molecular weight ranges are 1 kDa to 10 MDa, values outside these intervals being deliberately envisaged as well - for example when cleaving chromosomes, molecular weight ranges extend up to about 2 times 10⁵ MDa, for example in case of human chromosome 1.

In a further preferred embodiment, further means of cleaving at least one covalent bond of said at least one molecule are brought into contact with said at least one molecule, said further means preferably being an enzyme, a chemical and/or a catalyst. Chemicals include art-established chemicals such as acids (for example HCl, HNO₃, H₂SO₄, and HIO₄) and bases (such as NaOH and KOH). Useful catalysts, in addition to enzymes, include the following non-enzymatic catalysts: sulfonated carbonaceous surfaces, metal oxides, H-form zeolithes, metal ions such as Zn²⁺ and Mg2⁺.

While recognizing that the present invention provides for avoiding the means recited in this embodiment altogether, it is considered that for tailored or enhanced applications, a combination of the newly discovered "magnetically induced cleavage" with art-established means of cleavage may be beneficial.

In a more preferred embodiment, said enzyme is a protease in case of said molecule being a polypeptide, a protein or a peptide; a nuclease in case of said molecule being a nucleic acid or an oligonucleotide; a ribonuclease in case of said molecule being a ribonucleic acid or an oligoribonucleotide; and a saccharidase or glycosidase in case of said molecule being an oligosaccharide or a polysaccharide. Preferred proteases include trypsin, LysC, GluC, AspN, ArgC and chymotrypsin.

Of note, said molecule may comprise any combination of amino acids, nucleotides and saccharides. For example, it may contain both peptidic or proteinacous moieties as well as saccharides connected to said moieties e.g. by glycosidic bonds. Examples are glycosylated proteins. Fragmentation of glycosylated proteins may include or consist of the removal or fragmentation of the saccharides and/or fragmentation of the peptidic or proteinaceous moiety. To the extent fragmentation is to be improved or modulated by the above disclosed further means, inter alia glycosidases may be employed.

In another preferred embodiment, which may, but does not have to be combined with the above further means of cleaving, non-magnetic beads may be added to the reaction mixture, for example said solution or suspension. Such non-magnetic beads are distinct from the particles optionally comprised in the magnetic body, and furthermore distinct from paramagnetic beads as commonly used in analytics. The non-magnetic beads may be ceramic beads, polymer beads, glass beads, or metal beads, the metal being a non-magnetic metal. In terms of size, they are preferably in the range between 1 µm and 5 mm such as between 0.1 mm and 2 mm. Also preferred is that said non-magnetic particles have the same or a similar size range as compared to the size of the magnetic body or magnet.

Without wishing to be bound by a specific theory, such beads may exert shearing forces when moved around by action of the at least one magnetic body, which shearing forces in turn facilitate cleavage of said at least one covalent bond. Also, it is considered that the non-magnetic particles, by restricting the motion of the molecules to be cleaved, the probability of colliding of said molecules with the magnetic body (or, equivalently, the number of collisions per time unit) is increased. This increases the yield or performance of the method of the invention.

In a further preferred embodiment, an inert viscous liquid; and/or a gel such as a polyacrylamide gel or agarose gel is added to said sample. Without wishing to be bound by a specific theory, it is considered that by embedding the molecule(s) to be fragmented in a gel or a viscous liquid, is a means of enhancing energy transfer from the magnetic body or, to the extent present, any non-magnetic particle, to said molecule(s), which in turn enhances yield. In Example 1, use is made of agarose.

In a further preferred embodiment of the method of the first aspect of this invention, (a) the solution or suspension is a liquid sample such as a buffer or bodily fluid, and the molecule is a nucleic acid; (b) the solution or suspension is drinking water or pool water, and the molecule is a macromolecular contamination such as a toxin; (c) the solution or suspension is sewage, and the molecule is a biological molecule such as an antibiotic or peptide hormone; (d) the solution or suspension is food or beverage, and the molecule is gluten; (e) the solution or suspension comprises a protein, polypeptide and/or peptide, proteinaceous raw material for growth media and/or proteinaceous raw material for a personal care product such as a shampoo, conditioner or soap, and the molecule is said or a protein, polypeptide and/or peptide; (f) the solution or suspension comprises a macromolecular toxin, and the molecule is said macromolecular toxin; or (g) the solution or suspension comprises undesired enzymatic activity, and the molecule is the protein exhibiting said enzymatic activity.

These preferred embodiments illustrate how large-scale processes are amenable to improvement by the method of this invention.

In particular, in their art-established implementations, processes for reducing nucleic acid content generally require UV light or filtering. This can be avoided by the process of the present invention. It is understood that reducing nucleic acid content refers to long nucleic acids, in particular those which comprise coding sequences. By fragmenting such long nucleic acids with the method of the invention, shorter fragments are obtained, wherein by fine-tuning the method the average length of the fragments can be controlled. As opposed to long nucleic acids, the fragments usually can be tolerated in compositions such as buffers. Nucleic acids include DNA and RNA.

An exemplary field where nucleic acid contamination of reagents and buffers is undesirable is forensics. Example 1 demontrates fragmentation of DNA.

Providing drinking water and pool water meeting the established standards of hygiene and health security typically requires the use of aggressive chemicals such as chlorine or ozone. The present invention allows to dispense with the handling of such hazardous material.

Many toxins, in particular extremely toxic compounds such as botulinum toxin, diphtheria toxin, cholera toxin and ricin are of biological origin and proteinaceous in nature. Fragmenting these toxins by means of "magnetically induced proteolysis" in accordance with the invention is a convenient means of controlling the risk arising from the potential presence thereof in materials or compositions to be brought into contact with humans.

Related thereto are the following further aspects:
In a second aspect, this invention provides a method of reducing nucleic acid content in a buffer, said method comprising the method of the first aspect disclosed above, wherein said buffer is said solution or suspension. Preferably, said nucleic acid is a long nucleic acid, i.e., nucleic acid comprising one or more coding sequences.

In a third aspect, the invention provides a method of reducing undesired enzymatic activity present in a solution or suspension, said method comprising applying the method of the first aspect disclosed above to said solution or suspension comprising said enzymatic activity.

In a fourth aspect, the invention provides a method of detoxifying or sterilizing a solution or suspension comprising a macromolecular toxin, said method comprising applying the method of the first aspect to said solution or suspension comprising a macromolecular toxin.

Preferably, said methods of the second, third and fourth aspect are performed in continuous mode as defined further above.

In a fifth aspect, the invention provides a use of a magnetic body and means for generating a fluctuating or oscillating magnetic field for cleaving at least one covalent bond in at least one molecule.

Preferred embodiments of the method of the first aspect apply mutatis mutandis to the use of the fifth aspect.

In a sixth aspect, the invention provides a device comprising or consisting of: (a) an electric conductor, preferably at least one coil, more preferably a Helmholtz coil; (b) a vessel and/or an array of vessels such as a microtiter plate; and (c) at least one magnetic body in said vessel or at least one magnetic body per vessel in said array of vessels;
wherein elements (a) and (b) are configured such that said at least one magnetic body is under the influence of a magnetic field generated by said electric conductor when in use, preferably by implementing said conductor as a coil and by said vessel or said array of vessels being located within said coil.

Such device is tailored to the method of the first aspect. Of note, there is no requirement that a separate coil is placed in the proximity of each vessel or an array of vessel or that each of said vessels would be encircled by its own coil. Instead, a single coil of appropriate dimension to accommodate said array of vessels may be used.

Preferred in this context are the above disclosed Helmholtz coils which generate a homogenous magnetic field over extended spatial regions.

Preferred embodiments of the method of the first aspect, to the extent they provide details of the electric conductor, the vessel(s) and the magnetic body, apply *mutatis mutandis* to the device of the sixth aspect.

In a preferred embodiment of the device of the sixth aspect, said device further comprises or further consists of a control unit, said control unit being configured to perform the method of the first aspect.

In a particularly preferred embodiment, said control unit has a computer program product loaded which computer program product comprises instructions for performing the method of the first aspect.

In a related aspect, the invention provides a computer program product comprising instructions for performing the method of the first aspect.

In a preferred embodiment of the sixth aspect, said device is comprised in a liquid handling robot.

Related thereto, but in a separate aspect, the invention provides a liquid handling robot comprising the device of the sixth aspect.

In a seventh aspect, the invention provides the use of the device of the sixth aspect for performing the method of the first aspect.

In an eighth aspect, the invention provides a device comprising or consisting of: (a) an electric conductor, preferably at least one coil, more preferably a Helmholtz coil; (b) at least one vessel with at least two openings; and (c) at least one magnetic body, wherein elements (a), (b) and (c) are configured such that (i) said at least one magnetic body is under the influence of a magnetic field generated by said coil when in use; and (ii) liquid flowing through said tubular element when in use is in contact with said magnetic body.

Preferred embodiments of the method of the first aspect, to the extent they provide details of the electric conductor, the tubular element and the magnetic body, apply *mutatis mutandis* to the device of the eighth aspect.

For example, said device of the eighth aspect may comprise a filter, a mesh, a membrane, or a sponge holding said magnetic body; or a thread, a filament, a wire, or a spring connecting said magnetic body to said reactor, preferably without significantly restricting the motion of said magnetic body when said device is in use.

A particularly preferred embodiment of said device comprises a filter which is a molecular weight cut-off filter. Such filters retain all material which has a molecular weight above the cut-off while letting pass through all molecules with a molecular weight below the cut-off. By passing a sample in continuous mode through a such equipped device, fragments will pass the filter once their size is below the cut-off, wherein further fragmentation to yet smaller fragments will not occur.

This may be used to obtain a narrow distribution of fragment sizes.

In a ninth aspect, the invention provides the use of the device of the eighth aspect for performing the method of any one of the second, third and fourth aspect as well as aspects related thereto.

In a tenth aspect, the invention provides a kit comprising or consisting of (a) a vessel and/or an array of vessels; (b) at least one magnetic body, preferably at least one magnetic body per vessel; (c) an electric conductor; and (d) optionally a manual with instructions for assembling the device of the sixth aspect and/or for performing the method of the first aspect.

It is understood that any array of vessels, including art-established arrays such as microtiter plates may be used. Arrays may be two-dimensional (such as 8 times 12 and the known microtiter plates of higher density with, e.g., 384 and 1536 vessels) or one-dimensional (as referred to as "strips"), e.g. with 8 or 12 vessels.

Preferred embodiments of the method of the first aspect, to the extent they provide details of the electric conductor, the vessel(s) and the magnetic body, apply *mutatis mutandis* to the kit of the tenth aspect.

In a related aspect, a kit is provided which comprises a flow-through reactor such as a tubular element in place of the vessel or array of vessels of (a).

These kits provide those constituents which allow the user to assemble devices of the invention as disclosed further above. Also, the kits, owing to said constituents being provided separately, allow the user a certain degree of flexibility or to assemble a tailored device adapted to the requirements of a desired application of the present invention.

In a preferred embodiment, said kit further comprises or further consists of one or more buffers for preparing a solution or suspension as defined above.

In a further preferred embodiment, said kit further comprises or further consists of one or more selected from a protein, an enzyme, a chemical and a catalyst.

The Figures show:
- **Figure 1:**: First lane: 100bp DNA ladder. Left hand side: Yeast was boiled only. DNA remained in the pocket and did not migrate into the agarose gel. Right hand side: Yeast was treated with magnetic fragmentation. The signal in the pocket is reduced and a cloud of DNA fragments can be observed within the gel.
- **Figure 2:**: Marker: PageRuler™ Plus pre-stained protein ladder. "Fragmented": proteins treated by magnetic fragmentation. CA: Carbonic anhydrase. BSA: Bovine serum albumin. NIST: NIST human Antibody. GP: Glycogen phosphorylase.

The Examples illustrate the invention.

### Example 1

### Magnetic fragmentation of DNA

### Material

Fresh *S. cerevisiae* pellets containing 100 µg of proteins were used for all experiments. Cylindric 2 mm x 2 mm samarium cobalt magnets were used as magnetic body. Gel was stained with Midori Green from Nippon Genetics (MG10). 1x TAE was prepared by a dilution from 50x TAE (48.44 g Tris, 12.1 ml Acetic acid, 3.7224 g EDTA in 200 ml H₂O). 100 bp DNA ladder was used from Nippon Genetics (MWD100). 6x DNA loading buffer was prepared by mixing 0.2 g Cresol red (114472-5G) + 9 ml Glycerol (G6279-500ML) + 21 ml H₂O. Agarose for gel preparation was purchased from Nippon Genetics (Ag 01; #D00248).

### Methods

Agarose gels were prepared by diluting 1,2 g agarose in 60 ml 1xTAE by boiling. After solution cooled down, 5 µl Midori Green was added and small gel was poured. Sample preparation was always prepared with 100 µg yeast *S. cerevisiae* cells. Cells were solubilized in 100 µl H₂O, pH 7 and boil at 95 °C and 1000 rpm for 10 min. For magnetic fragmentation treatment a samarium cobalt magnet was added and cells were further incubated in a magnetic field of a frequency of 160 Hz at a magnetic flux density of about 1 mT for 4 h. 2 µl 6x loading dye were added to 10 µl of each sample and loaded onto the agarose gel. DNA ladder samples were directly loaded. Gel was run at 100 V for approximately 1 h.

### Results

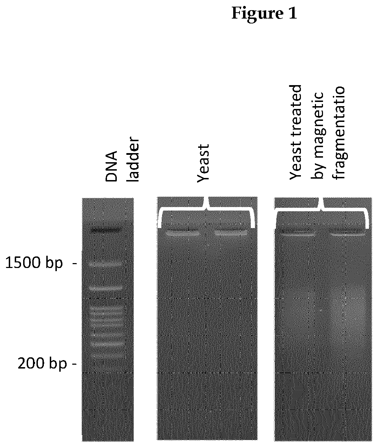

See Figure 1 and caption thereof.

### Discussion

Boiled but otherwise untreated yeast DNA is too large to migrate into the agarose gel as expected. The respective lane appears devoid of DNA fragments and therefore genomic DNA of yeast can be used to showcase DNA fragmentation. After treatment with magnetic fragmentation, a cloud appears in the DNA gel showcasing a range of fragments of various sizes between 200 and 1500 bp lengths.

### Example 2

### Magnetic fragmentation of proteins

### Material

Carbonic anhydrase (CA, sol.02.04.19), BSA (BioRad, 64124165), NIST mAb (14HBD-D-002, sol.16.02.19), and glycogen phosphorylase (GP, sol. 25.03.19) were used for fragmentation experiments. PageRuler ™ Plus prestained protein ladder, 10 to 250 kDa was used as marker (Thermo Scientific™, 11832124). Cylindric 3 mm x 3 mm NdFeB magnets were used as magnetic body. PAA gels were used for gel electrophoresis (BioRad Criterion Midi, 64316288). 4x Laemmli Sample Buffer (BioRad, 64172364) was used to prepare the sample for gel electrophoresis. 1x Running Buffer (25mM Tris, 0,1%SDS, 192mM Glycine; BioRad, 20200415) were used during electrophoresis. The gel was stained with Coomassie Brilliant Blue R-250 (BioRad 64105295). To destain the gel, 10% acetic acid (BioRad, 20200420) was used.

### Methods

100 µg protein at a concentration of 1 µg/µl in ultra-pure water were processed by magnetic fragmentation with a setting of 120Hz, at a magnetic flux density of about 1 mT in a single-coil set-up with 200 windings at 1cm inner diameter and for single Eppendorf 1.5mL vessels for 18 hours. 15µl of the solutions containing 15µg of protein were mixed with each 5µl of 4x loading dye (Laemmli buffer) and the total 20µl were loaded on a PAA gel. SDS-PAGE was performed at 200V for 40min. The gel was stained for 1h using Coomassie Brilliant Blue solution. Destaining was executed in a microwave at 440W with destaining solution, heating up for 10min, let cool down for 1 min and then rinsed with water. This cycle was repeated once.

### Results

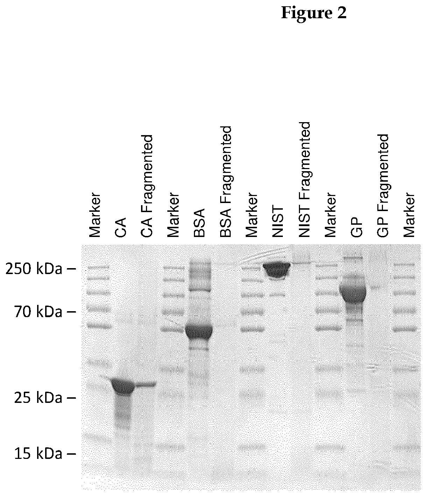

See Figure 2.

### Discussion

All proteins show clearly reduced signal with only minor signal remaining after treatment. This clearly indicates fragmentation and removal of pre-existing protein. The generated fragments are not clearly visible as expected as peptides are not well stained in SDS-PAGE.

## Claims

1. A method of cleaving at least one covalent bond in at least one molecule, wherein said method comprises
(a) colliding at least one magnetic body with said at least one molecule; and/or
(b) triggering collision of at least one non-magnetic particle with said at least one molecule by moving said at least one magnetic body.

2. The method of claim 1, wherein said at least one magnetic body performs a fluctuating or oscillating motion, wherein preferably said motion is triggered by a fluctuating or oscillating magnetic field, wherein preferably said magnetic field is generated by an electric current and/or an electromagnet.

3. The method of any claim 1 or 2, wherein said colliding transfers an amount of energy to said molecule which is sufficient to cleave said at least one covalent bond.

4. The method of any one of the preceding claims, wherein said at least one molecule is in solution or in suspension, and wherein preferably said at least one molecule and said at least one magnetic body are located in a reactor.

5. The method of any one of the preceding claims, wherein said method is performed in batch mode, e.g. by using a reactor which is a vessel with a closed bottom.

6. The method of claim 4, wherein said method is performed in continuous mode, e.g. said solution or suspension is allowed to flow over said at least one magnetic body, preferably
(a) in a reactor which has at least two openings; and/or
(b) wherein motion of said magnetic body occurs while said solution or suspension is flowing.

7. The method of any of claims 4 to 6, wherein the dimensions of said at least one magnetic body and said reactor are such that said at least one magnetic body moves freely or substantially freely about its average position, wherein preferably free or substantially free motion is around or along at least two, at least three, at least four, at least five or preferably all six axes of translational and rotational motion, and wherein preferably said free or substantially free motion includes at least translation along two axes.

8. The method of any one of the preceding claims, wherein said at least one molecule is a macromolecule or a chain molecule, wherein said macromolecule or chain molecule comprises or consists of at least two building blocks, wherein preferably said building blocks are amino acids, nucleotides, ribonucleotides, saccharides, phosphates, lipids, fatty acids, sugars, glycerides, or a combination of any of the foregoing.

9. The method of any one of the preceding claims, wherein further means of cleaving at least one covalent bond of said at least one molecule are brought into contact with said at least one molecule, said further means preferably being an enzyme, a chemical and/or a catalyst.

10. The method of any one of the preceding claims, wherein
(a) the solution or suspension is a liquid sample such as a buffer or a bodily fluid, and the molecule is a nucleic acid;
(b) the solution or suspension is drinking water or pool water, and the molecule is a macromolecular contamination;
(c) the solution or suspension is sewage, and the molecule is a biological macromolecule;
(d) the solution or suspension is food or beverage, and the molecule is gluten;
(e) the solution or suspension comprises a protein, polypeptide and/or peptide, proteinaceous raw material for growth media and/or proteinaceous raw material for a personal care product such as a shampoo, a conditioner or soap, and the molecule is said or a protein, polypeptide and/or peptide;
(f) the solution or suspension comprises a macromolecular toxin, and the molecule is said macromolecular toxin; or
(g) the solution or suspension comprises undesired enzymatic activity, and the molecule is the protein exhibiting said enzymatic activity.

11. Use of a magnetic body and means for generating a fluctuating or oscillating magnetic field for cleaving at least one covalent bond in at least one molecule.

12. A device comprising or consisting of:
(a) an electric conductor, preferably at least one coil, more preferably a Helmholtz coil;
(b) a vessel and/or an array of vessels such as a microtiter plate; and
(c) at least one magnetic body in said vessel or at least one magnetic body per vessel in said array of vessels;
wherein elements (a) and (b) are configured such that said at least one magnetic body is under the influence of a magnetic field generated by said electric conductor when in use, preferably by implementing said conductor as a coil and by said vessel or said array of vessels being located within said coil.

13. A liquid handling robot comprising the device of claim 12.

14. A device comprising or consisting of:
(a) an electric conductor, preferably at least one coil, more preferably a Helmholtz coil;
(b) at least one vessel with at least two openings; and
(c) at least one magnetic body,
wherein elements (a), (b) and (c) are configured such that
(i) said at least one magnetic body is under the influence of a magnetic field generated by said coil when in use; and
(ii) liquid flowing through said tubular element when in use is in contact with said magnetic body.

15. A kit comprising or consisting of
(a) a vessel and/or an array of vessels;
(b) at least one magnetic body, preferably at least one magnetic body per vessel;
(c) an electric conductor; and
(d) optionally a manual with instructions for assembling the device of claims 14 and/or for performing the method of any one of claims 1 to 10.
